(19) Europäisches Patentamt
European Patent Office
Office européen des brevets

(11) Veröffentlichungsnummer: **0 063 741**
**A1**

(12) # EUROPÄISCHE PATENTANMELDUNG

(21) Anmeldenummer: **82103115.0**

(22) Anmeldetag: **13.04.82**

(51) Int. Cl.³: **C 07 C 79/46**, C 07 C 103/48, A 01 N 37/48, A 01 N 43/40, A 01 N 43/48

(30) Priorität: **23.04.81 DE 3116205**

(43) Veröffentlichungstag der Anmeldung: **03.11.82 Patentblatt 82/44**

(84) Benannte Vertragsstaaten: **AT BE CH DE FR GB IT LI NL**

(71) Anmelder: **BAYER AG, Zentralbereich Patente, Marken und Lizenzen, D-5090 Leverkusen 1, Bayerwerk (DE)**

(72) Erfinder: **Förster, Heinz, Dr., Am Eckbusch 47, D-5600 Wuppertal 1 (DE)**
Erfinder: **Eue, Ludwig, Dr., Paul-Klee-Strasse 36, D-5090 Leverkusen 1 (DE)**
Erfinder: **Schmidt, Robert, Dr., Im Waldwinkel 110, D-5060 Bergisch-Gladbach 2 (DE)**
Erfinder: **Lürssen, Klaus, Dr., August-Kierspel-Strasse 89, D-5060 Bergisch-Gladbach 2 (DE)**

(54) **5-(2,6-Dichlor-4-trifluormethyl-phenoxy)-2-nitro-benzoesäure-Derivate, Verfahren zu ihrer Herstellung sowie ihre Verwendung als Herbizide und Pflanzenwachstumsregulatoren.**

(57) Neue 5-(2,6-Dichlor-4-trifluormethyl-phenoxy)-2-nitro-benzoesäure-Derivate der Formel

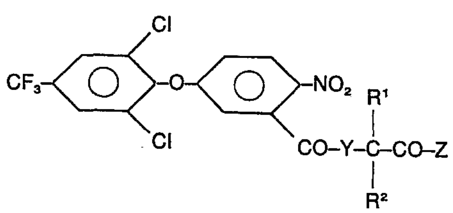

(I)

in welcher

$R^1$ und $R^2$ unabhängig voneinander für Wasserstoff oder Methyl stehen,

Y für Sauerstoff, Imino(N–H) oder Methylimino($N–CH_3$) steht und

Z für den Rest $OR^3$ steht, worin

$R^3$ für Wasserstoff, ein Natrium-, Kalium- oder Ammoniumkation, ein Magnesium- oder Calcium-Äquivalent, Methyl, durch Fluor, Chlor, Cyano, $C_1$–$C_4$-Alkoxy und/oder $C_1$–$C_4$-Alkylthio substituiertes Ethyl, für n- oder iso-Propyl, n-, iso-, sec- oder tert.-Butyl, $C_3$–$C_5$-Alkenyl, $C_3$–$C_5$-Alkinyl, Benzyl, Phenylethyl oder, – sofern Y für Sauerstoff oder Imino steht –, auch für Ethyl steht,

oder

Z für den Rest 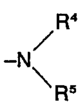 steht, worin

$R^4$ für Wasserstoff, für gegebenenfalls durch Fluor, Chlor, Cyano und/oder $C_1$–$C_4$-Alkoxy substituiertes $C_1$–$C_5$-Alkyl, für $C_3$–$C_5$-Alkenyl, $C_3$–$C_5$-Alkinyl, $C_3$–$C_6$-Cycloalkyl oder Benzyl steht, und

$R^5$ für Wasserstoff, für gegebenenfalls durch Fluor, Chlor, Cyano und/oder $C_1$–$C_4$-Alkoxy substituiertes $C_1$–$C_5$-Alkyl, für $C_1$–$C_5$-Alkoxy, $C_3$–$C_5$-Alkenyl, $C_3$–$C_5$-Alkinyl, $C_3$–$C_6$-Cycloalkyl, Benzyl, Phenylethyl oder Phenyl steht, welches ein- bis dreifach durch Methyl, Fluor, Chlor, Brom, Cyano, Nitro, Trifluormethyl und/oder Methoxy substituiert sein kann, oder worin

$R^4$ und $R^5$ zusammen mit dem Stickstoffatom, an das sie gebunden sind, für gegebenenfalls durch Methyl und/oder Ethyl substituiertes Pyrrolidyl, für gegebenenfalls durch Methyl substituiertes Morpholinyl, für Piperidyl, für 3-Methyl-, 4-Methyl-, 2-Ethyl-, 4-Ethyl-, 2,3-Dimethyl-, 2,4-Dimethyl-, 2,5-Dimethyl- oder 3,5-Dimethyl-piperidyl stehen oder, – sofern Y für Imino oder Methylimino steht –, auch für 2-Methylpiperidyl stehen,

mehrere Verfahren zu deren Herstellung sowie die Verwendung der neuen Stoffe als Herbizide und Pflanzenwachstumsregulatoren.

EP 0 063 741 A1

BAYER AKTIENGESELLSCHAFT 5090 Leverkusen, Bayerwerk
Zentralbereich
Patente, Marken und Lizenzen Dü/kl-c
Ib

# 5-(2,6-Dichlor-4-trifluormethyl-phenoxy)-2-nitro-benzoesäure-Derivate, Verfahren zu ihrer Herstellung sowie ihre Verwendung als Herbizide und Pflanzenwachstumsregulatoren

Die Erfindung betrifft neue 5-(2,6-Dichlor-4-trifluormethyl-phenoxy)-2-nitro-benzoesäure-Derivate, mehrere Verfahren zu ihrer Herstellung sowie ihre Verwendung als Herbizide und Pflanzenwachstumsregulatoren.

Es ist bekannt, daß bestimmte substituierte Diphenylether zur Unkrautbekämpfung verwendet werden können (vgl. R. Wegler, Chemie der Pflanzenschutz- und Schädlingsbekämpfungsmittel, Band 5, S. 73 - 80, Springer Verlag Berlin, Heidelberg, New York, 1977; ferner EP-A 20 052). Die Wirkung der als Herbizide vorbekannten Verbindungen aus der Reihe der Diphenylether ist jedoch für viele Zwecke nicht zufriedenstellend.

Es wurden nun neue 5-(2,6-Dichlor-4-trifluormethyl-phenoxy)-2-nitro-benzoesäure-Derivate der Formel

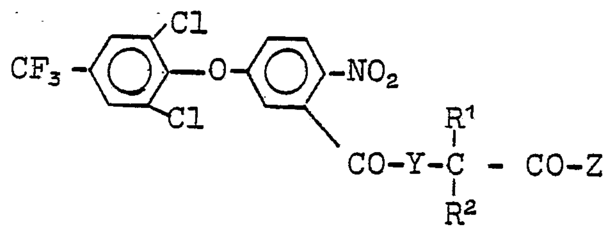

(I)

in welcher

$R^1$ und $R^2$ unabhängig voneinander für Wasserstoff oder Methyl stehen,

Y für Sauerstoff, Imino (N-H) oder Methylimino ($N-CH_3$) steht und

Z für den Rest $OR^3$ steht, worin

$R^3$ für Wasserstoff, Natrium, Kalium oder Ammonium, ein Magnesium- oder Calcium-Äquivalent, Methyl, durch Fluor, Chlor, Cyano, $C_1$-$C_4$-Alkoxy und/oder $C_1$-$C_4$-Alkylthio substituiertes Ethyl, für n- oder iso-Propyl, n-, iso-, sec- oder tert.-Butyl, $C_3$-$C_5$-Alkenyl, $C_3$-$C_5$-Alkinyl, Benzyl, Phenylethyl oder, - sofern Y für Sauerstoff oder Methylimino steht - , auch für Ethyl steht,

oder

Z für den Rest 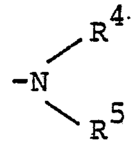 steht, worin

$R^4$ für Wasserstoff, für gegebenenfalls durch Fluor, Chlor, Cyano und/oder $C_1$-$C_4$-Alkoxy substituiertes $C_1$-$C_5$-Alkyl, für $C_3$-$C_5$-Alkenyl, $C_3$-$C_5$-Alkinyl, $C_3$-$C_6$-Cycloalkyl oder Benzyl steht, und

$R^5$ für Wasserstoff, für gegebenenfalls durch Fluor, Chlor, Cyano und/oder $C_1$-$C_4$-Alkoxy substituiertes $C_1$-$C_5$-Alkyl, für $C_1$-$C_5$-Alkoxy, $C_3$-$C_5$-Alkenyl, $C_3$-$C_5$-Alkinyl, $C_3$-$C_6$-Cycloalkyl, Benzyl, Phenylethyl oder Phenyl steht, welches ein- bis dreifach durch Methyl, Fluor, Chlor, Brom, Cyano, Nitro, Trifluormethyl und/oder Methoxy substituiert sein kann,

oder worin

$R^4$ und $R^5$ zusammen mit dem Stickstoffatom an das sie gebunden sind, für gegebenenfalls durch Methyl und/oder Ethyl substituiertes Pyrrolidyl, für gegebenenfalls durch Methyl substituiertes Morpholinyl, für Piperidyl, für 3-Methyl-, 4-Methyl-, 2-Ethyl-, 4-Ethyl-, 2,3-Dimethyl-, 2,4-Dimethyl-, 2,5-Dimethyl- oder 3,5-Dimethyl-piperidyl stehen oder , - sofern Y für Imino oder Methylimino steht - , auch für 2-Methylpiperidyl stehen,

gefunden.

Weiterhin wurde gefunden, daß man die neuen Verbindungen der Formel (I) erhält, wenn man

a) 5-(2,6-Dichlor-4-trifluormethyl-phenoxy)-2-nitrobenzoesäurechlorid der Formel

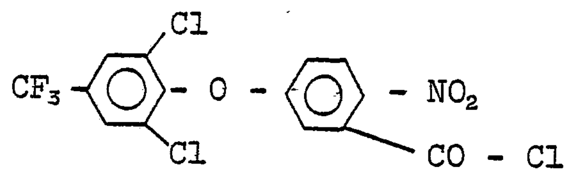

(II)

mit Verbindungen der Formel

$$HY - \underset{R^2}{\overset{R^1}{C}} - CO - Z \qquad (III)$$

in welcher

$R^1$, $R^2$, Y und Z die oben angegebene Bedeutung haben,

gegebenenfalls in Gegenwart eines Säureakzeptors und gegebenenfalls in Gegenwart eines Verdünnungsmittels umsetzt,

oder

b) 5-(2,6-Dichlor-4-trifluormethyl-phenoxy)-2-nitrobenzoesäure-Derivate der Formel

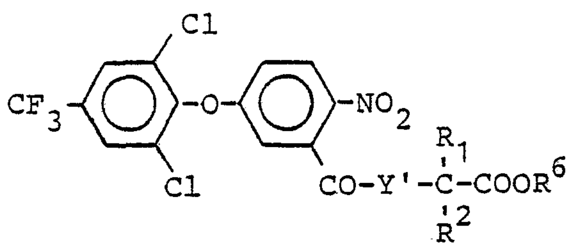

(Ia)

in welcher

$R^1$ und $R^2$ die oben angegebene Bedeutung haben,

Y' für Imino oder Methylimino steht und

$R^6$ für Methyl, Ethyl, n- oder iso-Propyl, n-, iso-, sec- oder tert.-Butyl steht,

mit Hilfe von Alkalihydroxid in Gegenwart eines Verdünnungsmittels verseift und anschließend mit einer starken Protonensäure ansäuert,

oder

c) 5-(2,6-Dichlor-4-trifluormethyl-phenoxy)-2-nitrobenzoesäure-Derivate der Formel

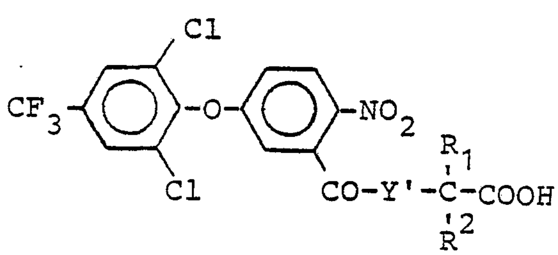

(Ib)

in welcher

$R^1$, $R^2$ und Y' die oben angegebene Bedeutung haben,

mit Natrium- bzw. Kaliumhydroxid oder Natriummethylat, gegebenenfalls in Gegenwart eines Natrium-, Kalium-, Ammonium-, Magnesium- oder Calciumsalzes in Gegenwart eines Verdünnungsmittels umsetzt,

oder

d) 3,5-Dichlor-4-hydroxy-benzotrifluorid der Formel

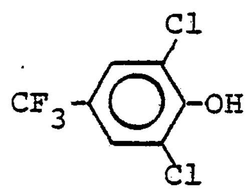

(IV)

mit 5-Halogen-2-nitro-benzoesäure-Derivaten der Formel

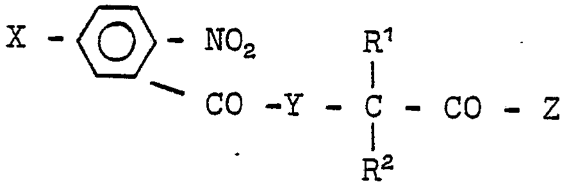

(V)

in welcher

$R^1$, $R^2$, Y und Z die oben angegebene Bedeutung haben und

X für Fluor, Chlor oder Brom steht,

gegebenenfalls in Gegenwart eines Säureazeptors sowie in Gegenwart eines Verdünnungsmittels umsetzt.

Schließlich wurde gefunden, daß sich die neuen 5-(2,6-Dichlor-4-trifluormethyl-phenoxy)-2-nitro-benzoesäure-Derivate der Formel (I) durch hervorragende herbizide und pflanzenwuchsregulierende Wirksamkeit auszeichnen. Die erfindungsgemäßen Wirkstoffe können insbesondere zur selektiven Bekämpfung von Unkräutern und Ungräsern in wichtigen Kulturen, wie z.B. Baumwolle, Getreide und Sojabohnen, eingesetzt werden. Die pflanzenwuchsregulierende Wirkung der neuen Stoffe kann vor allem in der Anwendung als Defoliants und Desiccants bei Baumwolle genutzt werden.

Überraschenderweise zeigen die erfindungsgemäßen 5-(2,6-Dichlor-4-trifluormethyl-phenoxy)-2-nitro-benzoesäure-Derivate der Formel (I) eine wesentlich bessere herbizide und pflanzenwuchsregulierende Wirksamkeit als aus dem Stand der Technik bekannte Verbindungen analoger Struktur und gleicher Wirkungsrichtung.

Verwendet man 5-(2,6-Dichlor-4-trifluormethyl-phenoxy)-2-nitro-benzoesäurechlorid und Methylaminoessigsäuredimethylamid als Ausgangsstoffe, so kann der Verlauf des erfindungsgemäßen Verfahrens (a) durch das folgende Formelschema wiedergegeben werden:

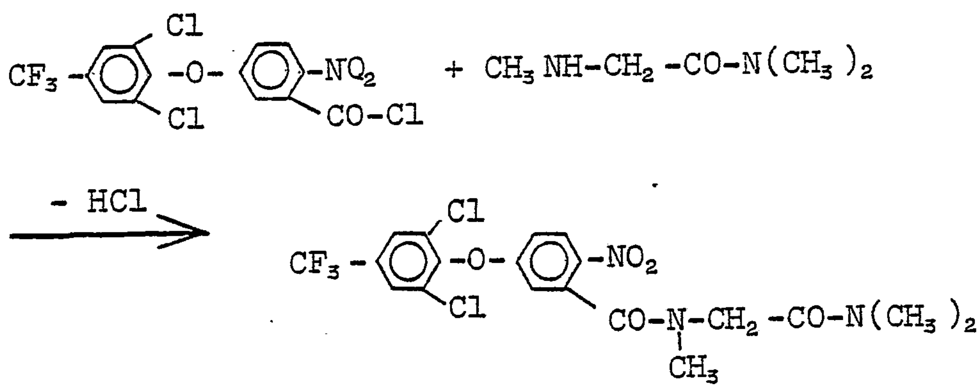


Verwendet man 5-(2,6-Dichlor-4-trifluormethyl-phenoxy)-2-nitro-benzoesäure-ethoxycarbonylmethylamid als Ausgangsstoff, Natriumhydroxid als Verseifungsmittel und Salzsäure als starke Protonensäure, so kann der Verlauf des erfindungsgemäßen Verfahrens (b) durch das folgende Formelschema wiedergegeben werden:

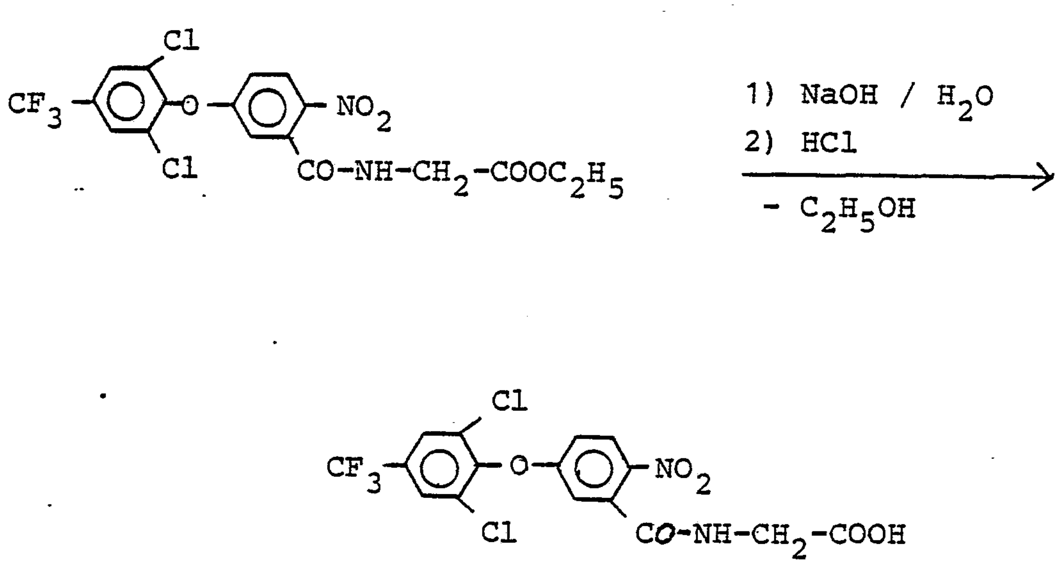

Verwendet man 5-(2,6-Dichlor-4-trifluormethyl-phenoxy)-2-nitro-benzoesäurehydroxycarbonylmethylamid als Ausgangsstoff und Kaliumhydroxid als Reaktionskomponente, so kann der Verlauf des erfindungsgemäßen Verfahrens (c) durch das folgende Formelschema wiedergegeben werden:

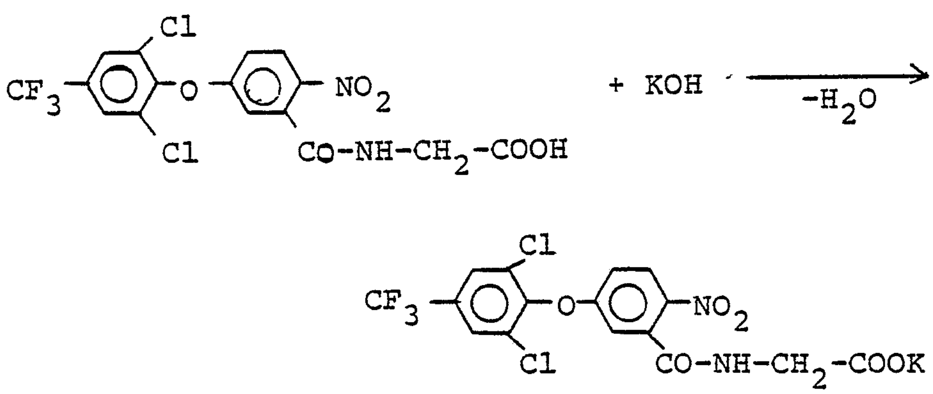


Verwendet man 3,5-Dichlor-4-hydroxy-benzotrifluorid und 4-Chlor-2-nitrobenzoesäure-(N,N-dimethylcarbonyl)-methylamid als Ausgangsstoffe, so kann der Verlauf des erfindungsgemäßen Verfahrens (d) durch das folgende Formelschema wiedergegeben werden:

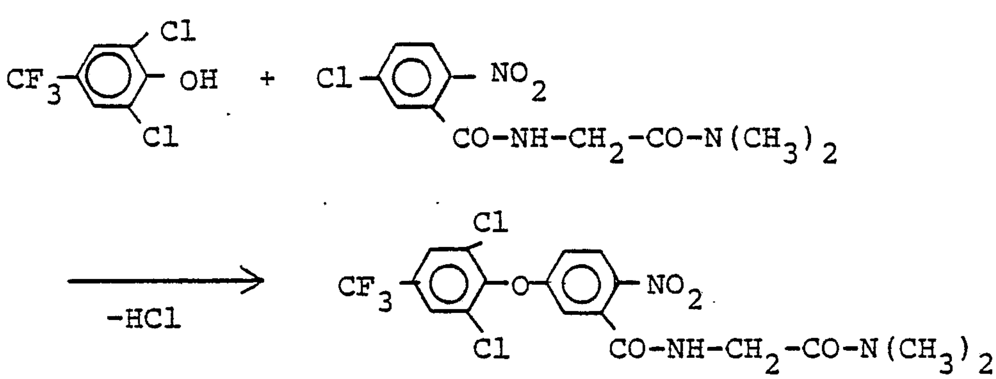

Das bei der Durchführung des erfindungsgemäßen Verfahrens (a) als Ausgangsprodukt benötigte 5-(2,6-Dichlor-4-trifluormethyl-phenoxy)-2-nitro-benzoesäurechlorid der Formel (II) ist bisher noch nicht bekannt. Man erhält diese Verbindung, wenn man 5-(2,6-Dichlor-4-trifluormethyl-phenoxy)-2-nitro-benzoesäure der Formel

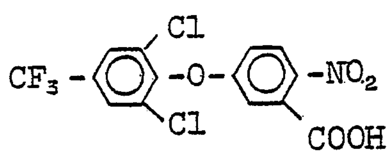

(VI)

mit einem Chlorierungsmittel, wie z.B. Thionylchlorid, gegebenenfalls unter Verwendung eines Katalysators, wie z.B. Dimethylformamid oder Pyridin, und gegebenenfalls unter Verwendung eines Verdünnungsmittels, wie z.B. 1,2-Dichlorethan, bei Temperaturen zwischen 10 und 100°C umsetzt und anschließend flüchtige Komponenten unter vermindertem Druck abdestilliert.

Die weiterhin bei dem erfindungsgemäßen Verfahren (a) als Ausgangsstoffe benötigten Verbindungen sind durch die Formel (III) definiert. In dieser Formel haben $R^1$, $R^2$, Y und Z diejenigen Bedeutungen, die bereits im Zusammenhang mit der Beschreibung der erfindungsgemäßen Stoffe der Formel (I) für diese Reste genannt wurden.

Als Beispiele für Verbindungen der Formel (III) seien im einzelnen genannt:

Hydroxyessigsäure- und Hydroxypropionsäure-methylester, -ethylester,-n-propylester, -iso-propylester,-n-butylester, -iso-butylester, sec-butylester, -2,2,2-trifluorethylester, -2-chlorethylester, -2-methoxyethylester, -2-ethoxyethylester, -2-methylthio-ethylester, -2-ethylthio-ethylester, -allylester und -propargylester, Hydroxyessigsäure- und Hydroxypropionsäureamid, -methylamid, -ethylamid, -n- und -iso-propylamid, -n-, -iso-, -sec- und tert-butylamid, -dimethylamid, -diethylamid, di-n-propylamid, -di-iso-propylamid,-di-n-butylamid, -di-iso-butylamid, -N-methyl-ethylamid, -N-methyl-n-propylamid, -N-methyl-iso-propylamid, -N-methyl-n-butylamid, -N-methyl-iso-butylamid, -N-methyl-sec-butylamid, -N-ethyl-n-propylamid, -N-ethyl-iso-butylamid, -N-methyl-sec.-butylamid, -N-ethyl-n-propylamid, -N-ethyl-n-butylamid, -N-propyl-n-butylamid, -N-propyl-iso-butylamid, -N-propyl-sec-butylamid, -diallylamid, -N-methylallylamid, -N-ethyl-allylamid, -N-propyl-allylamid, -N-iso-propyl-allylamid, -N-butyl-allylamid, -N-iso-butyl-allylamid, -N-sec-butyl-allylamid, -N-methyl-propargylamid, -N-ethyl-propargylamid, -N-propyl-propargylamid, -N-iso-butyl-propargylamid, -N-sec-butyl-propargylamid, -N-methyl-(1-methyl-propargyl)-amid, -dipropargylamid, -dicyclopentenylamid, -dicyclohexylamid, -N-methyl-cyclopentylamid, -N-methyl-cyclohexylamid, -N-ethyl-cyclopentylamid, -N-ethyl-cyclohexylamid, -dibenzylamid,

-N-methyl-benzylamid, -N-ethyl-benzylamid, -N-propylbenzylamid, -N-butyl-benzylamid, -N-allyl-benzylamid, -N-propargyl-benzylamid, -N-methyl-anilid, -N-ethylanilid, -N-propyl- anilid, -N-iso-propyl-anailid, -N-butyl-anilid, -N-isobutyl-anilid, -N-sec-butyl-anilid, -N-methyl-(2-methyl-phenyl)-amid, -N-methyl-(3-methyl-phenyl)-amid, -N-methyl-(4-methyl-phenyl)-amid, -N-methyl-(3-nitro-6-methyl-phenyl)-amid, -N-benzyl-anilid, -piperidid, -2-methyl, -3-methyl-, -4-methyl-, -2,4-dimethyl-, -2,4,6-trimethyl-, -2-ethyl-, -4-ethyl-, -2,4-diethyl-, -3,5-dimethyl-, -2-methyl-5-ethyl- und -2,4,6-triethyl-piperidid, -pyrrolidid, -2-methyl-pyrrolidid, -2,4-dimethyl-pyrrolidid, -2-methyl-5-ethyl-pyrrolidid, -morpholid, -3-methyl-morpholid und 3,5-dimethyl-morpholid, ferner Aminoessigsäure-, α-Aminopropionsäure und Methylaminoessigsäure-methylester, -ethylester, -n- und -iso-propylester sowie -n-, -iso-, -sec- und -tert-butylester.

Die Verbindungen der Formel (III) sind bekannt (vgl. DE-OS 29 04 490).

Bei der Durchführung des erfindungsgemäßen Verfahrens (a) arbeitet man vorzugsweise in Gegenwart eines Säureakzeptors. Als solche können alle üblichen Säurebindemittel Verwendung finden. Vorzugsweise in Frage kommen Alkali-hydroxide, wie z.B. Natrium- und Kaliumhydroxid, Alkalicarbonate und -alkoholate, wie Natrium- und Kaliumcarbonat, Natrium- und Kaliumethylat bzw. - methylat, ferner aliphatische, aromatische oder heterocyclische Amine, beispielsweise Triethylamin, Trimethylamin, Dimethylanilin, Dimethylbenzylamin und Pyridin.

Als Verdünnungsmittel können bei der Durchführung des erfindungsgemäßen Verfahrens (a) praktisch alle inerten organischen Solventien verwendet werden. Vorzugsweise in Betracht kommen aliphatische und aromatische, gegebenenfalls halogenierte Kohlenwasserstoffe, wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether, wie Diethylether und Dibutylether, Glycoldimethylether und Diglycoldimethylether, Tetrahydrofuran und Dioxan, Ketone, wie Aceton, Methyl-ethyl-, Methyl-isopropyl-, und Methyl-isobutylketon, Ester, wie Essigsäure-methylester und -ethylester, Nitrile, wie z.B. Acetonitril und Propionitril, Amide, wie z.B. Dimethylformamid, Dimethylacetamid und N-Methyl-pyrrolidon, sowie Dimethylsulfoxid, Tetramethylensulfon und Hexamethylphosphorsäuretriamid.

Die Reaktionstemperaturen können bei der Durchführung des erfindungsgemäßen Verfahrens (a) innerhalb eines größeren Bereiches variiert werden. Im allgemeinen arbeitet man bei Temperaturen zwischen -20°C und +100°C, vorzugsweise zwischen -10°C und +50°C.

Das erfindungsgemäße Verfahren (a) wird im allgemeinen bei Normaldruck ausgeführt.

Bei der Durchführung des erfindungsgemäßen Verfahrens (a) werden die Ausgangsstoffe der Formeln (II) und (III) im allgemeinen in etwa äquimolaren Mengen eingesetzt.

Ein Überschuß der einen oder anderen Komponente ist möglich. Die Umsetzung wird im allgemeinen in einem geeigneten Verdünnungsmittel in Gegenwart eines Säureakzeptors durchgeführt und das Reaktionsgemisch wird meherere Stunden bei der erforderlichen Temperatur gerührt. Danach gibt man ein organisches Lösungsmittel, z.B. Toluol, zu und arbeitet die organische Phase wie üblich durch Waschen, Trocknen und Abdestillieren des Lösungsmittels auf.

Die neuen Verbindungen fallen zum Teil in Form von Oelen an, die sich zum Teil nicht unzersetzt destillieren lassen, jedoch durch sogenanntes "Andestillieren", d.h. durch längeres Erhitzen unter vermindertem Druck auf mäßig erhöhte Temperatur von den letzten flüchtigen Anteilen befreit und auf diese Weise gereinigt werden. Zu ihrer Charakterisierung dient der Brechungsindex. Soweit die neuen Produkte in fester Form anfallen, können sie durch Umkristallisation gereinigt werden. Zur Charakterisierung dient dann der Schmelzpunkt.

Die bei dem erfindungemäßen Verfahren (b) als Ausgangsstoffe benötigten 5-(2,6-Dichlor-4-trifluormethylphenoxy)-2-nitro-benzoesäure-Derivate sind durch die Formel (Ia) definiert. Die betreffenden Verbindungen sind bisher noch nicht bekannt; sie lassen sich jedoch nach dem erfindungsgemäßen Verfahren (a) in einfacher Weise herstellen.

Als Alkalihydroxide, die bei dem erfindungsgemäßen Verfahren (b) als Reagenzien zur Verseifung der eingesetzten Ester benötigt werden, kommen vorzugsweise Natriumhydroxid und Kaliumhydroxid in Betracht.

Als Verdünnungsmittel kommen bei dem erfindungsgemäßen Verfahren (b) Gemische aus Wasser und organischen Lösungsmitteln, wie zum Beispiel Acetonitril, in Frage.

Als starke Protonensäuren kommen bei dem erfindungsgemäßen Verfahren (b) vorzugsweise Salzsäure oder wässrige Schwefelsäure in Betracht.

Die Reaktionstemperaturen können bei der Durchführung des erfindungsgemäßen Verfahrens (b) innerhalb eines größeren Temperaturbereiches variiert werden. Im allgemeinen arbeitet man bei Temperaturen zwischen 10° und 50°C.

Bei der Durchführung des erfindungsgemäßen Verfahrens (b) geht man im allgemeinen so vor, daß man die Verbindungen der Formel (Ia) in üblicher Weise verseift und anschließend das Reaktionsgemisch in überschüssige, wässrige Mineralsäure gießt. Dabei scheiden sich die Verbindungen der Formel (Ib) kristallin ab.

Die bei dem erfindungsgemäßen Verfahren (c) als Ausgangsstoffe benötigten 5-(2,6-Dichlor-4-trifluormethyl-phenoxy)-2-nitro-benzoesäure-Derivate sind durch die Formel (Ib) definiert. Die betreffenden Verbindungen

sind bisher noch nicht bekannt; sie lassen sich jedoch nach dem erfindungsgemäßen Verfahren (b) in einfacher Weise herstellen.

Als weitere Reaktionskomponenten dienen bei dem erfindungsgemäßen Verfahren (c) die Basen Natriumhydroxid, Natriummethylat oder Kaliumhydroxid, gegebenenfalls im Gemisch mit zusätzlich einem Natrium-, Kalium-, Ammonium-, Calcium- oder Magnesium-Salz, wobei Ammoniumchlorid, Calciumchlorid und Magnesiumchlorid beispielhaft genannt seien.

Als Verdünnungsmittel dient bei dem erfindungsgemäßen Verfahren (c) Wasser oder ein mit Wasser mischbares organisches Lösungsmittel, wie zum Beispiel Methanol. Es ist jedoch auch möglich, Gemische aus Wasser und einem mit Wasser mischbaren organischen Lösungsmittel beizusetzen.

Die Reaktionstemperaturen können bei der Durchführung des erfindungsgemäßen Verfahrens (c) innerhalb eines größeren Bereiches variiert werden. Im allgemeinen arbeitet man bei Temperaturen zwischen 0°C und 50°C, vorzugsweise bei Raumtemperatur.

Bei der Durchführung des erfindungsgemäßen Verfahrens (c) geht man im allgemeinen so vor, daß man die Verbindungen der Formel (Ib) mit den jeweils gewünschten Salzbildnern verrührt. Dabei scheiden sich die herzustellenden Produkte, gegebenenfalls nach vorherigem Einengen, in kristalliner Form ab.

Das bei dem erfindungsgemäßen Verfahren (d) als Ausgangsstoff benötigte 3,5-Dichlor-4-hydroxy-benzotrifluorid der Formel (IV) ist bekannt (vgl. DE-OS 2 016 624).

Die weiterhin bei dem erfindungsgemäßen Verfahren (d) als Ausgangsstoffe benötigten 5-Halogen-2-nitro-benzoesäure-Derivate sind durch die Formel (V) definiert. In dieser Formel haben $R^1$, $R^2$, Y und Z diejenigen Bedeutungen, die bereits im Zusammenhang mit der Beschreibung der erfindungsgemäßen Stoffe der Formel (I) für diese Reste genannt wurden. X steht vorzugsweise für Chlor oder Brom.

Die 5-Halogen-2-nitro-benzoesäure-Derivate der Formel (V) sind bisher noch nicht bekannt. Sie lassen sich jedoch nach üblichen Methoden herstellen. So erhält man Stoffe der Formel (V), indem man bekannte 5-Halogen-2-nitro-benzoesäure-chloride der Formel

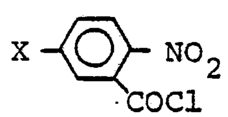

(VII)

in welcher

X die oben angegebene Bedeutung hat,

mit Verbindungen der Formel

$$HY-\underset{R^2}{\overset{R^1}{C}}-CO-Z \qquad \text{(III)}$$

in welcher

$R^1$, $R^2$, Y und Z die oben angegebene Bedeutung haben,

gegebenenfalls in Gegenwart eines Säureakzeptors sowie gegebenenfalls in Gegenwart eines Verdünnungsmittels umsetzt. Die Reaktionsbedingungen entsprechen dabei denjenigen, die bei der Umsetzung nach dem erfindungsgemäßen Verfahren (a) angewandt werden.

Als Säureakzeptoren können bei dem erfindungsgemäßen Verfahren (d) alle üblichen Säurebindemittel verwendet werden. Vorzugsweise in Frage kommen alle diejenigen Säurebinder, die im Zusammenhang mit der Beschreibung des erfindungsgemäßen Verfahrens (a) genannt wurden.

Als Verdünnungsmittel kommen bei dem erfindungsgemäßen Verfahrens (d) inerte organische Solventien, wie zum Beispiel Aceton und Acetonitril in Betracht.

Die Reaktionstemperaturen können bei der Durchführung des erfindungsgemäßen Verfahrens (d) innerhalb eines größeren Bereiches variiert werden. Im allgemeinen arbeitet man bei Temperaturen zwischen -20°C und +100°C, vorzugsweise zwischen -10°C und +50°C.

Die Umsetzung nach dem erfindungsgemäßen Verfahren (d) erfolgt unter den für derartige Reaktionen üblichen Bedingungen.

Die erfindungsgemäßen Wirkstoffe beeinflussen das Pflanzenwachstum und können deshalb als Defoliants, Desiccants, Krautabtötungsmittel, Keimhemmungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

Dicotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoelea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen z.B. Forst-, Ziergehölz-, Obst-, Wein-, Citrus-, Nuss-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe besitzen außerdem eine sehr gute pflanzenwachstumsregulierende Wirksamkeit. Sie eignen sich besonders gut zur Wuchshemmung, sowie zur Entlaubung und Austrocknung der Blätter bei Baumwolle.

Die Wirkstoffe können in die üblichen Formulierungen übergeführt werden, wie Lösungen, Emulsionen, Spritzpulver, Suspensionen, Pulver, Stäubemittel, lösliche Pulver, Granulate, Suspensions-Emulsionskonzentrate, Wirkstoffimprägnierte Natur- und synthetische Stoffe, Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole wie Butanol oder Glycol sowie deren Äther und Ester, Ketone, wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in

Frage: z.B. nichtionogene und anionische Emulgatoren, wie Polyoxyethylen-Fettsäure-Ester, Polyethylen-Fettalkohol-Ether, z.B. Alkylarylpolyglykol-ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azo - und Metallpthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischung möglich ist. Auch eine Mischung mit anderen bekannten Wirkstoffen, wie Fungiziden, Insektiziden, Akariziden, Nematiziden, Schutzstoffen gegen Vogelfraß, Wuchsstoffen, Pflanzennährstoffen und Bodenstrukturverbesserungsmitteln ist möglich.

Die Wirkstoffe können als solche, in Form ihrer Formulierungen oder der daraus durch weiteres Verdünnen bereiteten Anwendungsformen, wie gebrauchsfertige Lösungen, Suspensionen, Emulsionen, Pulver und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Die Anwendung wird vorzugsweise vor dem Auflaufen der Pflanzen, also im pre-emergence-Verfahren, vorgenommen. Sie können auch vor der Saat in den Boden eingearbeitet werden.

Die aufgewandte Wirkstoffmenge kann in größeren Bereichen schwanken. Sie hängt im wesentlichen von der Art des gewünschten Effektes ab. Im allgemeinen liegen die Aufwandmengen zwischen 0,05 und 10 kg Wirkstoff pro ha, vorzugsweise zwischen 0,1 und 5 kg/ha.

Die Herstellung und die Verwendung der erfindungsgemäßen Stoffe geht aus den nachfolgenden Beispielen hervor.

0063741

Herstellungsbeispiele

Beispiel 1

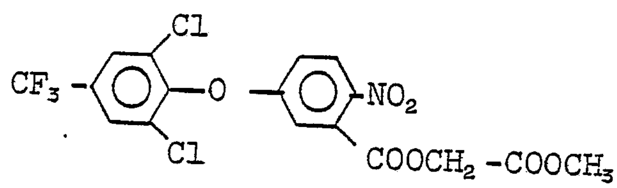

Eine Lösung von 29,7 g (76 mMol) 5-(2,6-Dichlor-4-trifluormethyl-phenoxy)-2-nitro-benzoesäurechlorid in 30 ml Aceton wird zu einer auf 0 bis 5°C gekühlten Lösung von 7,2 g (80 mMol) Glycolsäuremethylester und 8,1 g Triethylamin in 90 ml Aceton gegeben. Das Reaktionsgemisch wird 15 Stunden bei 20°C gerührt, dann in 1,5 l Wasser gegossen und mit Toluol extrahiert. Die organische Phase wird nacheinander mit Natronlauge und Wasser gewaschen, getrocknet und eingeengt. Der Rückstand wird mit Methanol verrührt und abgesaugt.
Man erhält 15,5 g (46 % der Theorie) 5-(2,6-Dichlor-4-trifluormethyl-phenoxy)-2-nitro-benzoesäure-methoxycarbonylmethylester vom Schmelzpunkt 119°C.

Beispiel 2

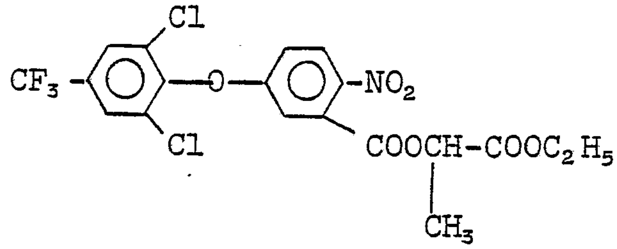

Eine Lösung von 29,7 g (76 mMol) 5-(2,6-Dichlor-4-trifluormethyl-phenoxy)-2-nitro-benzoesäurechlorid in 30 ml Aceton wird zu einer auf 0 bis 5°C gekühlten Lösung von 9,4 g (80 mMol) Milchsäureethylester und 8,1 g Triethylamin in 90 ml Aceton gegeben. Das Reaktionsgemisch wird 15 Stunden bei 20°C gerührt, dann in 1,5 l Wasser gegossen und mit Toluol extrahiert. Die organische Phase wird nacheinander mit Natronlauge und Wasser gewaschen, getrocknet und eingeengt. Man erhält 26,9 g (75 % der Theorie) 5-(2,6-Dichlor-4-trifluormethyl-phenoxy)-2-nitro-benzoesäure-(1-ethoxycarbonylethyl)-ester als Öl vom Brechungsindex $n_D^{20}$: 1,5334.

Beispiel 3

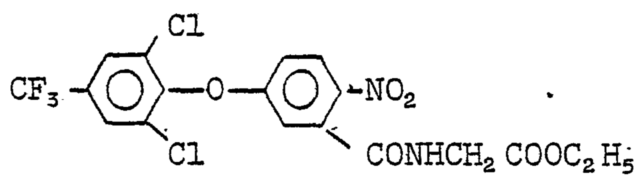

Zu einer Mischung aus 7 g (50 mMol) Aminoessigsäureethylester-hydrochlorid, 20,7 g (52 mMol) 5-(2,6-Dichlor-4-trifluormethyl-phenoxy)-2-nitro-benzoesäurechlorid und 180 ml Acetonitril werden bei -10 bis -5°C 4 g Natriumhydroxid, gelöst in 10 ml Wasser, gegeben. Nach Ende der Umsetzung wird das Reaktionsgemisch auf Wasser gegossen. Der pH-Wert wird auf 10 eingestellt und es wird vom kristallinen Produkt abgesaugt.

Man erhält 23g (95 % der Theorie) 5-(2,6-Dichlor-4-trifluormethyl-phenoxy)-2-nitro-benzoesäure-ethoxycarbonylmethylamid vom Schmelzpunkt 146°C.

Beispiel 4

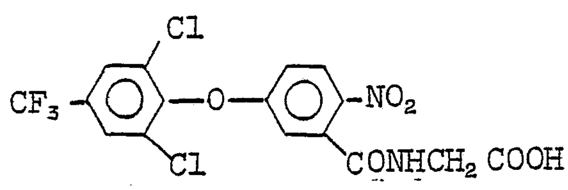

120,5 g 5-(2,6-Dichlor-4-trifluormethyl-phenoxy)-2-nitro-benzoesäure-ethoxycarbonylmethylamid (vgl. Beispiel 3) werden mit 10,5 g Natriumhydroxid in einem Gemisch aus 350 ml Acetonitril und 125 ml Wasser 90 Minuten bei 20°C gerührt. Das Reaktionsgemisch wird filtriert und das Filtrat wird zu 3 l 2n Salzsäure gegeben, wobei sich das Produkt kristallin abscheidet. Man erhält 106,7g (94 % der Theorie) 5-(2,6-Dichlor-4-trifluormethyl-phenoxy)-2-nitro-benzoesäure-hydroxycarbonylmethylamid vom Schmelzpunkt 219°C.

Beispiel 5

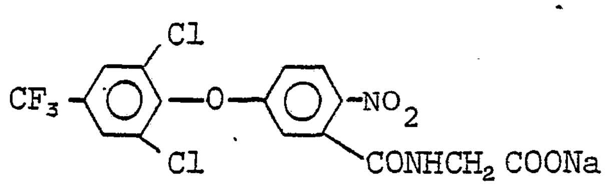

27,2g 5-(2,6-Dichlor-4-trifluormethyl-phenoxy)—2-nitro-benzoesäure-hydroxycarbonylmethylamid (vgl. Beispiel 4) werden zu einer Lösung von 3,3g Natriummethylat in 80 ml Methanol gegeben. Das Gemisch wird eine Stunde bei 20°C gerührt, filtriert und eingeengt. Man erhält als Rückstand 27,3g (96 % der Theorie) 5-(2,6-Dichlor-4-trifluormethylphenoxy)-2-nitro-benzoesäure-oxycarbonylmethylamid-natriumsalz.

Beispiel 6

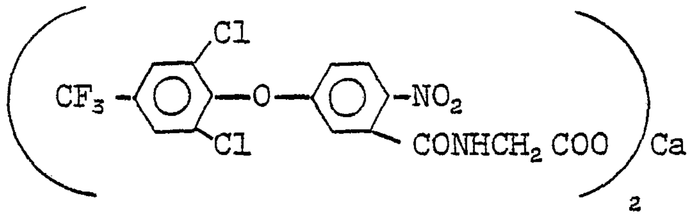

4,4g Calciumchlorid, gelöst in 25 ml Wasser, werden bei 20°C zu einer Mischung aus 27,2g 5-(2,6-Dichlor-4-trifluormethyl-phenoxy)-2-nitro-benzoesäure-hydroxycarbonylmethylamid (vgl. Beispiel 4), 2,5g Natriumhydroxid und 60 ml Wasser gegeben. Das Gemisch wird 15 Stunden bei 20°C gerührt und abgesaugt. Man erhält 31g (100 % der Theorie) 5-(2,6-Dichlor-4-trifluormethyl-phenoxy)-2-nitro-benzoesäure-oxycarbonylmethylamid-calciumsalz.

Nach den vorausgehend beschriebenen Methoden können die in der nachstehenden Tabelle aufgeführten Verbindungen hergestellt werden:

Tabelle 1

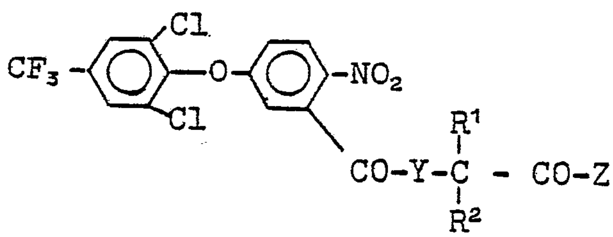

(I)

| Bsp. Nr. | Y | $R^1$ | $R^2$ | Z | Physikal. Konstante (Schmelzpunkt/ Brechungsindex) |
|---|---|---|---|---|---|
| 7 | O | H | H | $-OC_4H_9$-n | |
| 8 | O | H | H | $-OCH_2CF_3$ | |
| 9 | O | H | H | $-OC_3H_7$-i | |
| 10 | O | H | H | $-OCH_2CH_2Cl$ | |
| 11 | O | H | H | $-OCH_2CH_2OCH_3$ | |
| 12 | O | H | H | $-OCH_2CH_2SC_2H_5$ | |
| 13 | O | H | H | $-OCH_2C\equiv CH$ | |
| 14 | O | H | H | $-OCH_2CH=CH_2$ | |
| 15 | O | H | H | $-OC_4H_9$-n | |
| 16 | O | H | H | -OH | |
| 17 | O | H | H | -ONa | |
| 18 | O | H | H | $-OCa_{1/2}$ | |
| 19 | O | H | H | $-N(CH_3)_2$ | |
| 20 | O | H | H | $-N(C_2H_5)_2$ | |
| 21 | O | H | H | $-N(CH_2CH=CH_2)_2$ | |
| 22 | $NCH_3$ | H | H | $-OCH_3$ | |
| 23 | $NCH_3$ | H | H | $-OC_2H_5$ | |
| 24 | $NCH_3$ | H | H | $-OC_3H_7$-i | |

0063741

Tabelle 1 (Fortsetzung)

| Bsp. Nr. | Y | $R^1$ | $R^2$ | Z | Physikal.Konst. (Schmelzpunkt/ Brechungsindex) |
|---|---|---|---|---|---|
| 25 | $NCH_3$ | H | H | -OH | |
| 26 | $NCH_3$ | H | H | -ONa | |
| 27 | $NCH_3$ | H | H | $-OCa_{1/2}$ | |
| 28 | NH | H | $CH_3$ | $-OC_2H_5$ | |
| 29 | NH | H | $CH_3$ | $-OC_4H_9$-tert | |
| 30 | NH | H | $CH_3$ | -OH | |
| 31 | NH | H | $CH_3$ | -ONa | |
| 32 | NH | H | $CH_3$ | $-OCa_{1/2}$ | |
| 33 | O | H | H | 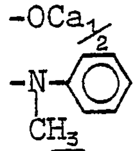 | |
| 34 | O | H | H | 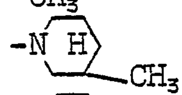 | |
| 35 | O | H | H | 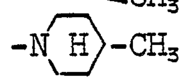 | |
| 36 | O | H | H | 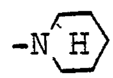 | |
| 37 | O | H | H | 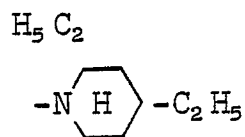 | |
| 38 | O | H | H | 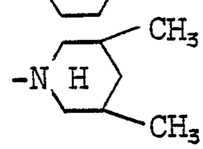 | |
| 39 | O | H | H | $-N(C_3H_7-n)_2$ | |
| 40 | O | H | H | 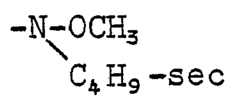 | |
| 41 | O | H | H | $-NH_2$ | |
| 42 | O | $CH_3$ | $CH_3$ | $-OC_2H_5$ | |

Herstellung von Ausgangsprodukten

Beispiel II

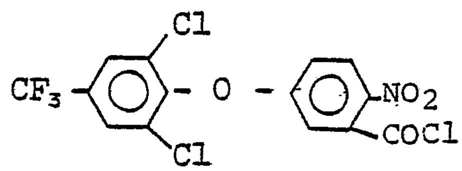

233 g Thionylchlorid und 4 ml Dimethylformamid werden zu einer Lösung von 650 g 5-(2,6-Dichlor-4-trifluormethyl-phenoxy)-2-nitro-benzoesäure in 1,5 l 1,2-Dichlorethan gegeben. Das Gemisch wird eine Stunde unter Rückfluß zum Sieden erhitzt. Danach wird das Lösungsmittel unter vermindertem Druck sorgfältig abdestilliert. Man erhält 5-(2,6-Dichlor-4-trifluormethyl-phenoxy)-2-nitro-benzoesäurechlorid als festen Rückstand mit einem Schemlzpunkt von 95°C.

Beispiel A

Pre-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoffzubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe (4), (5) und (6) eine sehr gute herbizide Wirksamkeit.

Beispiel B

Post-emergence-Test

Lösungsmittel: 5 Gewichtsteile Aceton
Emulgator: 1 Gewichtsteil Alkylarylpolyglycoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die in der Tabelle angegebenen Wirkstoffmengen pro Flächeneinheit ausgebracht werden. Die Konzentration der Spritzbrühe wird so gewählt, daß in 2000 l Wasser/ha die in der Tabelle angegebenen Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

In diesem Test zeigen die erfindungsgemäßen Wirkstoffe (4), (5) und (6) eine sehr gute herbizide Wirksamkeit.

Beispiel C

Entlaubung und Austrocknung der Blätter bei Baumwolle

Lösungsmittel: 30 Gewichtsteile Dimethylformamid
Emulgator : 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des 5.Folgeblattes angezogen. In diesem Stadium werden die Pflanzen tropfnaß mit den Wirkstoffzubereitungen besprüht. Nach 1 Woche werden der Blattfall und das Austrocknen der Blätter im Vergleich zu den Kontrollpflanzen bonitiert. Es bedeuten:

0 kein Austrocknen der Blätter, kein Blattfall
\+ leichtes Austrocknen der Blätter,geringer Blattfall
++ starkes Austrocknen der Blätter,starker Blattfall
+++ sehr starkes Austrocknen der Blätter, sehr starker Blattfall

In diesem Test zeigen die erfindungsgemäßen Wirkstoffe (1), (2) und (4) eine starke Wirksamkeit.

Patentansprüche

1) 5-(2,6-Dichlor-4-trifluormethyl-phenoxy)-2-nitro-benzoesäure-Derivate der Formel

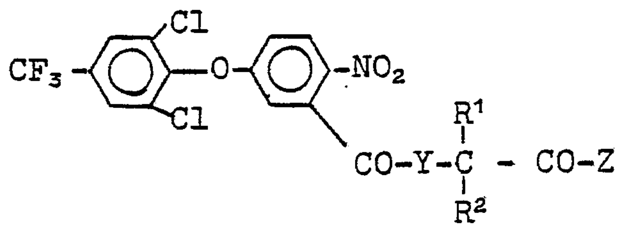

(I)

in welcher

$R^1$ und $R^2$ unabhängig voneinander für Wasserstoff oder Methyl stehen,

Y für Sauerstoff, Imino(N-H) oder Methylimino($N-CH_3$) steht und

Z für den Rest $OR^3$ steht, worin

$R^3$ für Wasserstoff, Natrium, Kalium oder Ammonium, ein Magnesium- oder Calciumionen-Äquivalent, Methyl, durch Fluor, Chlor, Cyano, $C_1$-$C_4$-Alkoxy und/oder $C_1$-$C_4$-Alkylthio substituiertes Ethyl, für n- oder iso-Propyl, n-, iso-, sec- oder tert-Butyl, $C_3$-$C_5$-Alkenyl, $C_3$-$C_5$-Alkinyl, Benzyl, Phenylethyl oder sofern Y für Sauerstoff oder Methylimino steht, auch für Ethyl steht,

oder

Z für den Rest 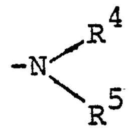 steht, worin

$R^4$ für Wasserstoff, für gegebenenfalls durch Fluor, Chlor, Cyano und/oder $C_1$-$C_4$-Alkoxy substituiertes $C_1$-$C_5$-Alkyl, für $C_3$-$C_5$-Alkenyl, $C_3$-$C_5$-Alkinyl, $C_3$-$C_6$-Cycloalkyl oder Benzyl steht,

und

$R^5$ für Wasserstoff, für gegebenenfalls durch Fluor, Chlor, Cyano und/oder $C_1$-$C_4$-Alkoxy substituiertes $C_1$-$C_5$-Alkyl, für $C_1$-$C_5$-Alkoxy, $C_3$-$C_5$-Alkenyl, $C_3$-$C_5$-Alkinyl, $C_3$-$C_6$-Cycloalkyl, Benzyl, Phenylethyl oder Phenyl steht, welches ein- bis dreifach durch Methyl, Fluor, Chlor, Brom, Cyano, Nitro, Trifluormethyl und/oder Methoxy substituiert sein kann,

oder worin

$R^4$ und $R^5$ zusammen mit dem Stickstoffatom, an das sie gebunden sind, für gegebenenfalls durch Methyl und/oder Ethyl substituiertes Pyrrolidyl, für gegebenenfalls durch Methyl substituiertes Morpholinyl, für Piperidyl, für 3-Methyl-, 4-Methyl-, 2-Ethyl-, 4-Ethyl-, 2,3-Dimethyl-, 2,4-Dimethyl, 2,5-Dimethyl- oder 3,5-Dimethyl-piperidyl stehen oder, - sofern Y für Imino oder Methylimino steht-, auch für 2-Methylpiperidyl stehen.

2) Verfahren zur Herstellung von 5-(2,6-Dichlor-4-trifluormethyl-phenoxy)-2-nitro-benzoesäure-Derivaten der Formel

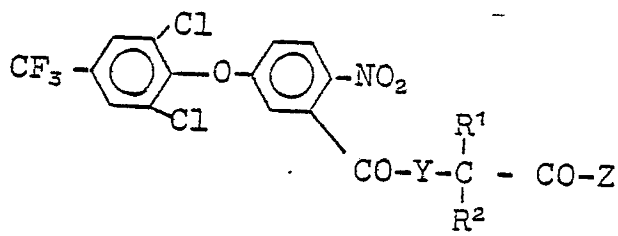

(I)

in welcher

$R^1$ und $R^2$ unabhängig voneiander für Wasserstoff oder Methyl stehen,

Y für Sauerstoff, Imino(N-H) oder Methyl-imino-($N-CH_3$) steht und

Z für den Rest $OR^3$ steht, worin

$R^3$ für Wasserstoff, Natrium, Kalium oder Ammonium, ein Magnesium- oder Calciumionen-Äquivalent, Methyl, durch Fluor, Chlor, Cyano, $C_1$-$C_4$-Alkoxy und/oder $C_1$-$C_4$-Alkylthio substituiertes Ethyl, für n- oder iso-Propyl, n-, iso-, sec- oder tert-Butyl, $C_3$-$C_5$-Alkenyl, $C_3$-$C_5$-Alkinyl, Benzyl, Phenylethyl oder ,- sofern Y für Sauerstoff oder Methylimino steht, auch für Ethyl steht,

oder

Z für den Rest $-N\begin{matrix}R^4\\R^5\end{matrix}$ steht, worin

$R^4$ für Wasserstoff, für gegebenenfalls durch Fluor, Chlor, Cyano und/oder $C_1$-$C_4$-Alkoxy substituiertes $C_1$-$C_5$-Alkyl, für $C_3$-$C_5$-Alkenyl, $C_3$-$C_5$-Alkinyl, $C_3$-$C_6$-Cycloalkyl oder Benzyl steht,

und

$R^5$ für Wasserstoff, für gegebenenfalls durch Fluor, Chlor, Cyano und/oder $C_1$-$C_4$-Alkoxy substituiertes $C_1$-$C_5$-Alkyl, für $C_1$-$C_5$-Alkoxy, $C_3$-$C_5$-Alkenyl, $C_3$-$C_5$-Alkinyl, $C_3$-$C_6$-Cycloalkyl, Benzyl, Phenylethyl oder Phenyl steht, welches ein- bis dreifach durch Methyl, Fluor, Chlor, Brom, Cyano, Nitro, Trifluormethyl und/oder Methoxy substituiert sein kann,

oder worin

$R^4$ und $R^5$ zusammen mit dem Stickstoffatom, an das sie gebunden sind, für gegebenenfalls durch Methyl und/oder Ethyl substituiertes Pyrrolidyl, für gegebenenfalls durch Methyl substituiertes Morpholinyl, für Piperidyl, für 3-Methyl-, 4-Methyl-, 2-Ethyl-, 4-Ethyl-, 2,3-Dimethyl-, 2,4-Dimethyl, 2,5-Dimethyl- oder 3,5-Dimethyl-piperidyl stehen oder, -sofern Y für Imino oder Methylimino steht-, auch für 2-Methylpiperidyl stehen,

dadurch gekennzeichnet, daß man

a) 5-(2,6-Dichlor-4-trifluormethyl-phenoxy)-2-nitro-benzoesäurechlorid der Formel

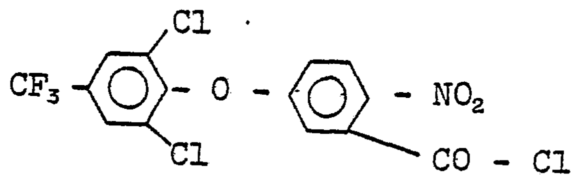

(II)

mit Verbindungen der Formel

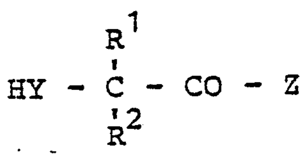

(III)

in welcher

$R^1$, $R^2$, Y und Z die oben angegebene Bedeutungen haben,

gegebenenfalls in Gegenwart eines Säureakzeptors und gegebenenfalls in Gegenwart eines Verdünnungsmittels umsetzt,

oder

b) 5-(2,6-Dichlor-4-trifluormethyl-phenoxy)-2-nitro-benzoesäure-Derivate der Formel

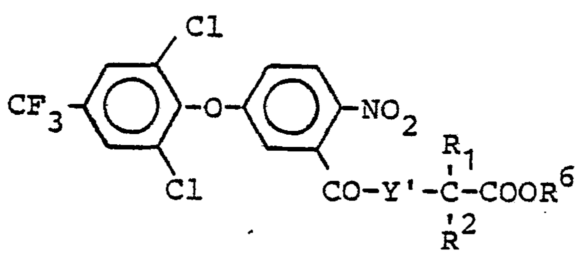

(Ia)

in welcher

$R^1$ und $R^2$ die oben angegebene Bedeutung haben,

Y' für Imino oder Methylimino steht

und

$R^6$ für Methyl, Ethyl, n- oder iso-Propyl, n-, iso-, sec- oder tert.-Butyl steht,

mit Hilfe von Alkalihydroxid in Gegenwart eines Verdünnungsmittels verseift und anschließend mit einer starken Protonensäure ansäuert,

oder

c) 5-(2,6-Dichlor-4-trifluormethyl-phenoxy)-2-nitrobenzoesäure-Derivate der Formel

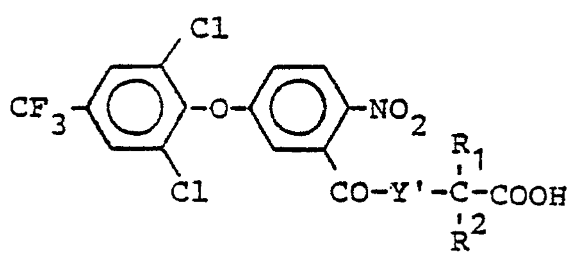

(Ib)

in welcher

$R^1$, $R^2$ und Y' die oben angegebene Bedeutung haben,

mit Natrium- bzw. Kaliumhydroxid oder Natriummethylat gegebenenfalls in Gegenwart eines Natrium-, Kalium-, Ammonium-, Magnesium- oder Calciumsalzes in Gegenwart eines Verdünnungsmittels umsetzt,

oder

d) 3,5-Dichlor-4-hydroxy-benzotrifluorid der Formel

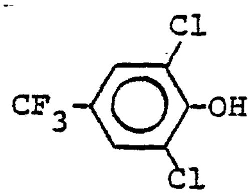

(IV)

mit 5-Halogen-2-nitro-benzoesäure-Derivaten der Formel

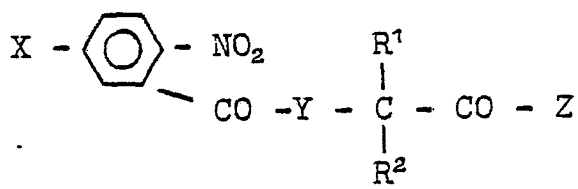

(V)

in welcher

$R^1$, $R^2$, Y und Z die oben angegebene Bedeutung haben und

X für Fluor, Chlor oder Brom steht,

gegebenenfalls in Gegenwart eines Säureakzeptors sowie in Gegenwart eines Verdünnungsmittels umsetzt.

3) Herbizide und pflanzenwuchsregulierende Mittel, gekennzeichnet durch einen Gehalt an mindestens einem 5-(2,6-Dichlor-4-trifluormethyl-phenoxy)-2-nitro-benzoesäure-Derivat der Formel (I).

4) Verfahren zur Bekämpfung von Unkräutern sowie zur Regulierung des Pflanzenwachstums, dadurch gekennzeichnet, daß man 5-(2,6-Dichlor-4-trifluormethyl-phenoxy)-2-nitro-benzoesäure-Derivate der Formel (I) auf Unkräuter bzw. die zu regulierenden Pflanzen und/oder deren Lebensraum aufbringt.

5) Verwendung von 5-(2,6-Dichlor-4-trifluormethyl-phenoxy)-2-nitro-benzoesäure-Derivaten der Formel (I) zur Bekämpfung von Unkräutern sowie zur Regulierung des Pflanzenwachstums.

6) Verfahren zur Herstellung von herbiziden bzw. pflanzenwuchsregulierenden Mitteln, dadurch gekennzeichnet, daß man 5-(2,6-Dichlor-4-trifluormethyl-phenoxy)-2-nitro-benzoesäure-Derivate der Formel (I) mit Streckmitteln und/oder oberflächenaktiven Stoffen vermischt.

7) 5-(2,6-Dichlor-4-trifluormethyl-phenoxy)-2-nitro-benzoesäure-Derivat der Formel

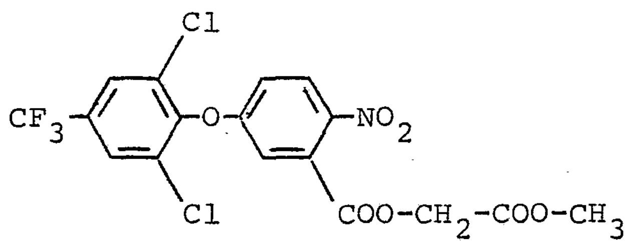

8) 5-(2,6-Dichlor-4-trifluormethyl-phenoxy)-2-nitro-benzoesäure-Derivat der Formel

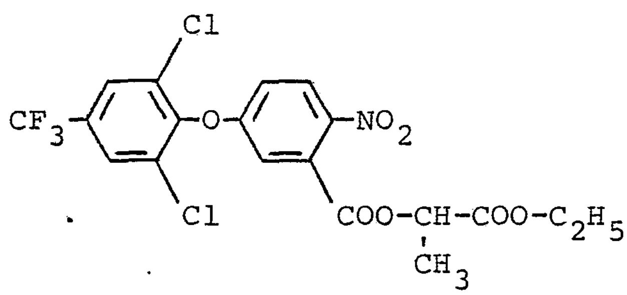

9) 5-(2,6-Dichlor-4-trifluormethyl-phenoxy)-2-nitro-benzoesäure-Derivat der Formel

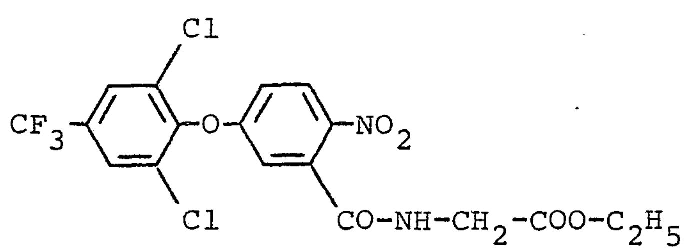

10) 5-(2,6-Dichlor-4-trifluormethyl-phenoxy)-2-nitrobenzoesäure-Derivat der Formel

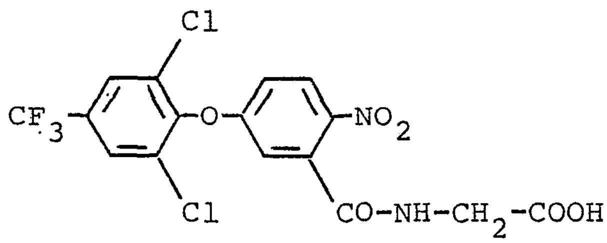

Europäisches Patentamt

# EUROPÄISCHER RECHERCHENBERICHT

0063741
Nummer der Anmeldung

EP 82 10 3115

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl. 3) |
|---|---|---|---|
| X | EP-A-0 020 052 (ROHM AND HAAS) * Seite 2, Zeile 5 - Seite 4, Zeile 6; Seite 8, Zeile 8 - Seite 10, Zeile 7; Seiten 54-55; Beispiele * --- | 1,2-6 | C 07 C 79/46<br>C 07 C 103/48<br>A 01 N 37/48<br>A 01 N 43/40<br>A 01 N 43/48 |
| P,E | EP-A-0 040 898 (MOBIL OIL) * Seite 1, Abschnitt 3; Seite 15, Anspruch 1; Seite 16, Anspruche 14,15; Seite 17, Anspruche 16-21 * --- | 1-6 | |
| X | GB-A-2 058 055 (PPG INDUSTRIES) * Seiten 7,8; Anspruche 1-4,11,14; Seite 1, Zeile 52 * ----- | 1-6 | |
| | | | RECHERCHIERTE SACHGEBIETE (Int. Cl. 3) |
| | | | C 07 C 69/00<br>C 07 C 79/00<br>C 07 C 67/00 |

Der vorliegende Recherchenbericht wurde für alle Patentansprüche erstellt.

| Recherchenort | Abschlußdatum der Recherche | Prüfer |
|---|---|---|
| DEN HAAG | 02-07-1982 | KINZINGER J.M. |

KATEGORIE DER GENANNTEN DOKUMENTEN
X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument